# EUROPEAN PATENT SPECIFICATION

(11) **EP 3 381 935 B1**
(45) Date of publication and mention of the grant of the patent: **25.03.2020**
(21) Application number: 18472002.7
(22) Date of filing: 20.02.2018
(51) Int. Cl.: C07K 14/57

(54) **ANTI-GAMMA MUTANT PROTEIN AGAINST ENDOGENOUS HUMAN INTERFERON- GAMMA**
HEMMSTOFF VON ENDOGENEM MENSCHLICHEM INTERFERON GAMMA
INHIBITEUR DE L' INTERFERON-GAMMA HUMAIN ENDOGENE

(30) Priority: 29.03.2017 BG 11247917
(43) Date of publication of application: 03.10.2018
(73) Proprietor: Tigo GmbH, 65193 Wiesbaden (DE)
(72) Inventor: TILEVA, Milena H., 1231 Sofia (BG); KRACHMAROVA, Elena B., 1000 Sofia (BG); NATCHEVA, Genoveva A., 1113 Sofia (BG); MASKOS, Klaus, D-94060 Pocking (DE); IVANOV, Ivan G., 1517 Sofia (BG); GRIGOLEIT, Patricia, 65193 Wiesbaden (DE)
(74) Representative: Stefanova, Stanislava Hristova

(56) References cited:
- WO-A1-2006/099701
- WO-A1-2009/124362
- US-A1- 2008 260 820
- NACHEVA G ET AL: "Human interferon gamma: Significance of the C-terminal flexible domain for its biological activity", ARCHIVES OF BIOCHEMISTRY AND BIOPHYSICS, ACADEMIC PRESS, US, vol. 413, no. 1, 1 May 2003 (2003-05-01), pages 91-98, XP002373770, ISSN: 0003-9861, DOI: 10.1016/S0003-9861(03)00113-9
- EALICK S E ET AL: "THREE-DIMENSIONAL STRUCTURE OF RECOMBINANT HUMAN INTERFERON-GAMMA", SCIENCE, AMERICAN ASSOCIATION FOR THE ADVANCEMENT OF SCIENCE, vol. 252, 3 May 1991 (1991-05-03), pages 698-702, XP002027561, ISSN: 0036-8075, DOI: 10.1126/SCIENCE.1902591

## Description

### Field of Invention

The present invention relates to an anti-gamma mutant protein against endogenous human interferon-gamma (hIFN-γ) applicable as therapeutic for curing of autoimmune diseases, associated with overexpression of endogenous hIFN-γ.

### Background of Invention

Autoimmune diseases occur when the body's immune system begins to produce antibodies (autoimmune antibodies) against a body protein and attacks by mistake the healthy tissue (respectively organ) where the target protein is localized. There are more than 80 different autoimmune diseases, leading to disability and early death of about 2% of the human population. Some of the common autoimmune diseases are rheumatoid arthritis, Hashimoto's thyroiditis, lupus erythematosus, multiple sclerosis, neurodegenerative diseases, some types of infertility in men and women, etc. Hence, the demand of therapeutics for specific suppression of autoimmunity is one of the priority tasks of experimental medicine and pharmacy.

Many studies have shown that pathogenesis of most autoimmune diseases is related to overproduction of proinflammatory cytokines, and particularly of human interferon-gamma (hIFN-γ), which is known to be an aggravating factor in the course of these diseases. Inflammation reaction accompanying the autoimmune process is related to a lavish infiltration of the target tissue with T-lymphocytes and macrophages. They are represented by CD4+ cells producing Th1 proinflammatory cytokines such as interleukin 12 (IL-12) and hIFN-γ. The latter stimulate mononuclear cells to produce destructive substances such as lymphotoxins (LT) and tumor necrosis factor alpha (TNF-α). It has been found that the overproduction of hIFN-γ plays a key role in the pathogenesis of many autoimmune diseases [1-3]. Different approaches have been developed for decreasing the level of endogenous hIFNγ in the case of multiple sclerosis (MS). In fact, the commonly used for treatment of MS hIFN-β suppresses the overproduction of hIFN-γ. Several patents such as US082138, WO9530435 arid CA2361081 have been developed on that basis, whereas other patents (RU2073522, RU2187332, RU02166959) recommend mixtures of the three different interferons (IFN-α, IFN-β and IFN-γ). There are reports showing that the high dosage of IFN-β (8,000,000 IU daily) result in adverse effects such as T-cells proliferation blockade [4], neutralization of IL-12 thus enhancing the hIFN-γ effect [5], decreased content of CD4+ and CD8+ cell without changing the Th1/Th2 cell ratio [6], decreased levels of both pro- and anti- inflammatory cytokines [7], etc.

Accordingly, in both periodic [8, 9] and patent (WO0145747) literature another therapeutic approach is described consisting of neutralization of the endogenous hIFN-γ by specific humanized monoclonal anti-hIFN-γ antibodies ("antigamma therapy"). The anti-hIFN-γ antibodies, however, deprive the organism from the vital for the body hIFN-γ. Thus, the prolonged administration of neutralizing anti-hIFN-γ antibodies worsens the patients' health and accordingly that approach has been shelved.

An alternative way for neutralizing the overexpressed hIFN-γ in case of autoimmune diseases is based on the application of the so called "consensus interferons" (IFN-con₁, IFN-con₂ and IFN-con₃) derived from the three interferons hIFN-α, hIFN-β and hIFN-τ (US0086534 and CA2299361). They have also demonstrated various adverse health effects including toxicity.

Recombinant hIFN-γ analogues bearing partial amino acid sequence of the hIFN-γ have been applied as antiviral, antitumor, antineoplastic or immunomodulating agents (US4832959, WO02081507, AT393690). Their clinical effects, however, cannot be assessed since the cited patents are not supported by clinical data.

Patent application, published as WO2006/099701A1, describes a new approach for inhibition of the endogenous hIFN-γ using inactive recombinant analogues of the hIFN-γ with preserved affinity to the hIFN-γ receptor. Subject of the patent application are three different inactive variants of hIFN-γ: truncated hIFN-γ lacking its C-terminal amino acids; fusion hIFN-γ-hIFN-α1 protein and an UV inactivated hIFN-γ. The recombinant proteins thus obtained cannot trigger the interferon-gamma induced intracellular signal transduction pathway and therefore they are capable of competing with the endogenous hIFN-γ for the hIFN-γ receptor.

In this view, according to patent application WO2009/124362A1, different hIFN-γ derivatives are obtained by site directed mutagenesis of the hIFN-γ gene to substitute amino acids at positions 86, 87 and 88, which are known to play a key role in the hIFN-γ mediated signal transduction. Since the receptor binding sites of hIFN-γ are located mainly at the N-terminus, the mutations do not affect the spatial structure, of hIFN-γ in general and therefore they are capable of binding to the receptor. The patent application reveals two gene-modified versions of hIFN-γ: mutant K88Q (with glutamine substitution for lysine in position 88) and C-terminally truncated K88Q with partially or completely removed unstructured C-terminus. The molecules of the two hIFN-γ mutants consist of 143 and 122 amino acids respectively. What is claimed is that the both proteins are potential agents for treatment of autoimmune diseases such as multiple sclerosis (MS), alopecia areata (AA) and myastenia gravis (MG).

### Description

The present invention relates to an anti-gamma mutant protein that is an inactive-hIFN-γ structural analogue with preserved binding affinity to the hIFN-γ receptor but deprived of ability to trigger the hIFN-γ-specific signal transduction pathway. In particular, the anti-gamma mutant protein represents a molecule in which the last five C-terminal amino acid (**aa**) residues are removed, the **aa** at position 88 is substituted with glutamine (Q) and the **aa** at position 27 is replaced with tyrosine (Y) or the **aa** at position 41 is replaced with asparagine (N). The two anti-gamma mutant proteins consisting of 138 **aa** are denoted as K88Q_T27Y (SEQ ID NO: 1) and K88Q_D41N (SEQ ID NO: 2) respectively. The shorter sequence length is chosen on the base of the fact that in the blood stream hIFN-γ undergoes processing which generates shorter and more stable variants of hIFN-γ with a preserved biological activity [10].

To our understanding the competition between the active (hIFN-γ) and inactive (anti-gamma mutant) proteins for the hIFN-γ receptor resembles the competitive enzyme inhibition. Therefore, the effect of that inhibition will depend on the relative concentrations of the two competitors, which outlines new therapeutic perspective for the treatment of autoimmune diseases.

The structure of the two anti-gamma mutant proteins, according to the present invention, is designed on the base of the 3D structure of hIFN-γ [11], on the 3D structure of the complex hIFN-γ-receptor [12], as well as on the base of an own computer model representing the electrostatic interaction of hIFN-γ with its receptor [13].

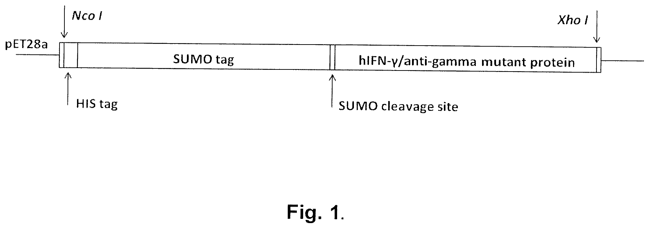

Codon optimized synthetic genes, encoding the two anti-gamma mutant proteins (SEQ ID NO:1 and SEQ ID NO:2) were synthesized by Life Technologies and expressed in *E. coli* cells by the SUMO fusion technology. To this end, the hIFN-γ mutant genes were fused to other partner sequences and were cloned into the expression vector pET28a. The expression cassettes thus constructed code for a hybrid protein consisting of an N-terminal hexahistidine tag (HIS tag) and a SUMO motif followed by the corresponding anti-gamma mutant protein (Fig. 1). The HIS-SUMO tag assists in protein folding, protein purification and removal of the HIS-SUMO tag by a SUMO specific protease. The complete removal of HIS-SUMO tag, together with the N-terminal methionine by SUMO protease is confirmed by MS fingerprinting. Thus the SUMO fusion technology results in production of methionine free recombinant proteins, which is a great advantage, considering that in some cases the residual N-terminal methionine might cause immunogenic response [14]. The procedure is described in details in [15].

The fusion HIS-SUMO-tagged proteins are expressed in *E. coli* BL21 (DE3)/pG-KJE8 cells, co-expressing the *E. coli* chaperone systems DnaK-DnaJ-GrpE and GroEL-GroES. Due to that the target proteins are properly folded *in vivo* and remain soluble in bacterial cytoplasm.

The recombinant proteins thus obtained are isolated by a two-step purification procedure including: 1) affinity chromatography on a Ni-column (to isolate the HIS-SUMO tag containing hybrid protein) and 2) ion-exchange chromatography (to purify the target protein from the HIS-SUMO tag). (Described in details in [15]). The purified anti-gamma mutant proteins were tested for hIFN-γ residual biological activity by the kynurenine assay [16] using three controls: 1) standard hIFN-γ; 2) C-terminally truncated by 5 aa hIFN-γ consisting of 138 aa; 3) C-terminally truncated by 5 aa hIFN-γ consisting of 138 aa plus a point mutation denoted K88Q. Table 1 below presents test results for the specific activity.

**Table 1. Biological activity of anti-gamma mutant proteins**

| **Recombinant protein** | **Specific activity, IU/mg** |
|---|---|
| anti-gamma mutant protein K88Q_T27Y (138 aa) | 4 x 10⁴ |
| anti-gamma mutant protein K88Q_D41 N (138 aa) | 4 x 10³ |
| Control 1: | 2-5 x 10⁷ |
| reference hIFN-γ (143 aa) | |
| Control 2: | 3 x 10⁷ |
| truncated reference hIFN-γ (138 aa) | |
| Control 3: | 3 x 10⁵ |
| K88Q (138 aa) | |

As shown in Table 1, the residual specific activity of the two anti-gamma mutant proteins ranges from 4 x 10³ IU/mg (for K88Q_D41N) to 4 x 10⁴ IU/mg (for K88Q_T27Y), i.e. 7500 to 750 times lower than that of the standard hIFN-γ. Therefore the anti-gamma mutant protein K88Q_D41N might be considered inactive. Both proteins K88Q_T27Y and K88Q_D41N have significantly lower residual activity compared to the control hIFN-γ mutant K88Q.

Two different methods are used to measure the binding affinity of the purified anti-gamma mutant proteins to the hIFN-γ receptor: a) isothermal titration calorimetry (ITC); and b) surface plasmon resonance (SPR). In both cases a recombinant hIFN-γ receptor (the extracellular part of the IFNGR1) expressed in animal cells is used but in ITC experiments the hIFN-γ receptor is dissolved in buffer and in the case of SPR the receptor is immobilized on a sensory microchip.

ITC is a powerful method for characterization of the full thermodynamic profile and measuring enthalpy of molecular interactions. Thermodynamic profile includes the following parameters: binding free energy (ΔG⁰), entropy (ΔS⁰), enthalpy (ΔH⁰), number of binding sites (n), and binding constant (K_{A}). During the ITC experiments IFNGR1 is titrated with either anti-gamma mutant proteins or control (standard hIFN-γ) samples. Binding affinity and stability of the molecular complexes thus formed are measured and the thermodynamic characteristics of the ligand-receptor complexes are compared.

The SPR method also allows determination of energetic parameters of the hIFN-γ-IFNGR1 and anti-gamma mutant protein-IFNGR1 complex formation, such as the association (kₒₙ) and dissociation (k_{off}) constants, affinity constant (K_{A}) and dissociation constant (K_{D}). In the SPR experiments, the recombinant IFNGR1 receptor is immobilized on the surface of a sensor chip, and the tested anti-gamma mutant proteins or hIFN-γ are let through the chip at a steady flow. Table 2 below presents the obtained results.

**Table 2. Thermodynamic parameters of ligand-receptor complexes formed between IFNGR1 and hIFN-γ or anti-gamma mutant proteins**

| **Recombinant protein** | **K_{D}, (nM) measured by iTC** | **K_{A}, (nM) measured by** |
|---|---|---|
| K88Q_T27Y | 28.2 ± 7.9 | 142 |
| K88Q_D41N* | - | 78.7 |
| Control 1: hIFN-γ | 136 ± 23.0 | 30.8 |
| Control 2: K88Q | 18.9 ± 2.8 | 30.6 |

| | | |
|---|---|---|
| * The anti-gamma mutant protein K88Q_D41N is measured by SPR method only | | |

As shown in the Table, the binding constant (K_{A}) of the mutant protein K88Q (control 2) as measured by SPR is comparable with that of the hIFN-γ (control 1), whereas the K_{A} of the anti-gamma mutant proteins K88Q_T27Y and K88Q_D41 N are 2-3 times higher than those of the hIFN-γ and K88Q. This indicates undoubtedly that the affinity of the two anti-gamma mutants K88Q_T27Y and K88Q_D41N to the hIFN-γ receptor is extremely high. In the iTC experiments, the dissociation constants (K_{D}) of K88Q and K88Q_T27Y are 4-6 times lower than that of the standard hIFN-γ. These data are in agreement with the data obtained by SPR and demonstrate that both anti-gamma proteins K88Q_T27Y and K88Q_D41N bind more tightly the hIFN-γ receptor compared to the reference hIFN-γ. It should be highlighted that the preserved or increased affinity of the mutant proteins towards the IFN-γ receptor is a prerequisite for their use as IFN-γ antagonists in the treatment of diseases associated with overexpression of hIFN-γ.

Purified anti-gamma mutant proteins K88Q_T27Y and K88Q_D41N are further estimated for their ability to compete with reference hIFN-γ in a receptor- binding assay based on the human amniotic WISH cell line, which is extremely rich in hIFN-γ receptors [16]. To this end, the anti-gamma mutant proteins are mixed in different molar ratios with standard hIFN-γ. Biological activity of the mixtures is measured by kynurenine bioassay using pure hIFN-γ as a control. The ability of the two anti-gamma . mutant proteins K88Q_T27Y and K88Q_D41N, and also of the K88Q mutant (used as a control) to compete for the hIFN-γ receptor was evaluated by the reduction of the activity of the reference hIFN-γ. The obtained results are presented in Table 3 below.

**Table 3. Competitive bioassay of anti-gamma mutant proteins**

| **Anti-gamma mutant protein** | **Competitive ability %** |
|---|---|
| K88Q_T27Y | 35 |
| K88Q_D41N | 30 |
| Control: K88Q | 25 |

The data in Table 3 show that the anti-gamma mutant proteins compete effectively with hIFN-γ for the hIFN-γ receptor and according to their competitive capacity, they are ranked in the following order: K88Q_T27Y> K88Q_D41N> K88Q. The best results obtained with K88Q_T27Y can be explained by the additional mutation at the N-terminus at position 27. K88Q_D41N has an amino acid substitution at position 41 to stabilize its dimeric form in the complex with the receptor. Table 3 shows that in terms of competitiveness this protein is ordered between K88Q_T27Y and K88Q.

The advantages of the anti-gamma mutant proteins according to the invention cover several aspects: they are devoid of N-terminal methionine which is known to be responsible for the immunogenicity of some recombinant proteins [14]; the endotoxin content satisfies the requirements of the Food and Drug Administration (FDA); their affinity to the hIFN-γ receptor is greater than that of the reference hIFN-γ and also is higher than that of the previously synthesized anti-gamma mutants; the anti-gamma mutant proteins K88Q_T27Y and K88Q_D41N compete with the wild type hIFN-γ most efficiently at ratios in the range of 0.1:1 to 0.5:1, although the individual optimal drug concentration have to be determined prior treatment; both anti-gamma mutant proteins K88Q_T27Y and K88Q_D41N have lower residual hIFN-γ activity.

Based on the above described properties, the conclusion is that the anti-gamma mutant proteins K88Q_T27Y and K88Q_D41N are reliable competitors of the wild type hIEN-γ. Therefore they must be considered as perspective drug-candidates for treatment of patients suffering of autoimmune diseases related with increased levels of the endogenous hIFN-γ such as multiple sclerosis, alopecia areata, myasthenia gravis, rheumatoid arthritis, systemic lupus erythematosus, glomerulonephritis, Addison's disease, Graves's disease, Hashimoto's disease, Guillain-Barre syndrome, transplant atherosclerosis leading to rejection of the grafted organ, Alzheimer's disease, infertility, asthma, inflammatory bowel disease, Crohn's disease, Behter's syndrome, etc.

### Brief description of the figures

**Figure 1** represents the structure of the expression vector pET28a containing HIS-SUMO-hIFN-γ/ mutant where:
pET28a - vector for inducible expression;
*Nco* I - recognition site of the restriction endonuclease *Nco* I;
HIS, SUMO - coding sequences for HIS-SUMO "tag";
SUMO cleavage site - Protease cleavage site;
hIFN-γ/ mutant - coding sequence for either hIFN-γ or anti-gamma mutant protein;
*Xho* I - recognition site of the restriction endonuclease *Xho* I*;*

The following examples illustrate the present invention without limiting its scope and spirit.

### Example 1. Structure of the anti-gamma mutant proteins K88Q_T27Y and K88Q_D41N.

The primary structures of the anti-gamma mutant proteins are shown in the Sequence Listing (SEQ ID NO:1 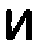 SEQ ID NO:2). Both K88Q_T27Y and K88Q_D41N anti-gamma mutants have 5 aa truncated C-terminus and bear the amino acid substitution Lys⁸⁸→Gln. The latter is designed to disrupt the integrity of the internal NLS signal sequence and therefore to deprive the proteins from their capacity to trigger the signal transduction pathway. In addition, the two proteins carry a second mutation, whose role is either to favor the interaction with the hIFN-γ receptor (as in K88Q_T27Y) or to stabilize the dimeric structure in the protein-receptor complex (as in K88Q_D41N). The two proteins are truncated by 5 C-terminal amino acids (i.e. consist of 138 amino acids) in order to achieve high stability and prolonged half-life in the blood stream [10].

According to the present invention, each anti-gamma mutant protein carries only two altered amino acids compared with the wild type hIFN=γ (resembling allelic variants), which is a prerequisite for the lack of immunogenicity when using as therapeutic agents.

### Example 2. Preparation and purification of the anti-gamma mutant proteins.

Codon optimized synthetic gene for protein expression encoding anti-gamma mutant protein K88Q_T27Y is synthesized by Life Technologies. A hybrid gene encoding fusion protein with an N-terminal 6-histidine tag (HIS), SUMO protein (SUMO) and the anti-gamma mutant protein K88Q_T27Y is generated by a two-step PCR. The hybrid gene carries two restriction sites (*Nco* I and *Xho* I) for cloning into the expression vector pET28a (Fig. 1). The DNA sequence thus created is verified by DNA sequencing. All cloning steps as well as the introduction of the hybrid gene into the expression vector pET28a are described in [15]. The expression plasmid is used for transformation of *E. coli* BL21(DE3)/pG-KJE8 cells (for details see ref. [15]). A similar approach is applied also for cloning and expression of the anti-gamma mutant protein K88Q_D41N.

The two anti-gamma mutant proteins are expressed in soluble form and purified by a two-step purification procedure. The first step includes isolation of the hybrid protein from clear cell lysates by Ni-affinity chromatography, and the second step consists in removal of the HIS-SUMO tag by specific proteolysis with SUMO-protease, and purification of the target proteins by ion-exchange chromatography. The N-terminal sequence structure is confirmed by protein sequencing. The improved purification procedure [15] is characterized by low cost and high reproducibility. The two proteins were tested for the presence of bacterial endotoxins and the results showed that they satisfied the requirements of FDA.

The yield and purity of the anti-gamma mutant proteins are presented in Table 4.

**Table 4. Yield and purity of the anti-gamma mutant proteins K88Q_T27Y and K88Q_D41N**

| **Anti-gamma mutant protein** | **Yield ^{a}, mg/g wet cell mass** | **Purity^{b}, %** |
|---|---|---|
| K88Q_T27Y | 5 | >98 |
| K88Q_D41N | 3 | >98 |

| | | |
|---|---|---|
| ^{a} Protein concentration was determined by Bradford ^{b} Protein purity was estimated by PAGE | | |

### Example 3. Examination of anti-gamma mutant proteins for hIFN-γ suppressor activity.

Purified anti-gamma mutant proteins prepared according to the invention are examined for hIFN-γ suppressor activity using human amniotic cells. (WISH). The cells are incubated in the presence of mixtures of different molar ratios of anti-gamma protein and wild type hIFN-γ (used as a standard). The suppressor activity of the mutants is evaluated by the decrease in antiproliferative activity of the wild type hIFN-γ measured by a kynurenine bioassay [16].

Experimentally the suppressor activity of the anti-gamma mutant proteins is determined as follows: The tested protein is serially diluted and samples of 50 µl are mixed with 50 µl of standard hIFN-γ in PVC 96 well microplates. WISH cell suspension (50 µl) in MEM Eagle medium supplemented with HEPES, L- glutamine and 2% BFS is added, mixed with 50 µl L-tryptophan and the kynurenine bioassay is performed according to Ref. [16]. Samples containing standard hIFN-γ (only) are used as positive control in this assay. Absorption at 490 nm (OD₄₉₀) is measured in an ELISA microplate reader and the average value for a given sample is compared with that of the cells treated with standard hIFN-γ only.

The data presented in Table 3 show that both anti-gamma mutant proteins K88Q_T27Y and K88Q_D41N compete with the wild type hIFN-γ to a greater degree than the single mutant K88Q. The two anti-gamma proteins are most effective at ratios of anti-gamma mutant protein to standard hIFN-γ of 0.1:1 to 0.5:1. The low hIFN-γ inhibitory concentration of the mutants is of great advantage for the development of drugs for autoimmune diseases.

### Example 4. Examination of a binding affinity of anti-gamma mutant proteins to the interferon-gamma receptor (IFNGR1) by isothermal microcalorimetry (iTC).

The thermodynamic profile and enthalpy of interaction of hIFN-γ and anti-gamma mutant proteins with the soluble (extracellular) part of the hIFN-γ receptor IFNGR1 are measured on a MicroCal Auto-iTC200 instrument. Soluble part of hIFN-γ receptor IFNGR1 is obtained by secretion expression of a synthetic IFNGR1 gene in insect cells and purification by a two-step chromatography procedure. All recombinant proteins (IFNGR1, anti-gamma mutant proteins and the wild type hIFN-γ) are extensively dialyzed against 20 mM HEPES pH 8.0 and 0.15 M NaCl before titration. Purified IFNGR1 (5-15 µM) is placed in the calorimetric cell and titrated with reference hIFN-γ or anti-gamma mutant protein (40-150 µM) by successive injections of 2 µL, and measurements are performed at 20°C with constant agitation speed of 750 rpm. The experimental data are processed by a MicroCal Origin software package.

The obtained results shown in Table 2 demonstrate that all ligand-receptor interactions are exothermic and that the affinity of anti-gamma mutant proteins to the interferon-gamma receptor (Kd = 19-28 nM) is greater (up to 5 times) than that of the reference hIFN-γ (Kd = 136 nM).

All these results indicate that the two anti-gamma mutant proteins K88Q_T27Y and K88Q are extremely effective competitors of hIFN-γ for its cellular receptor.

**Example 5.** Examination of a binding affinity of anti-gamma mutant proteins to the interferon-gamma receptor (IFNGR1) by surface plasmon resonance (SPR). To this end biotinylated recombinant hIFN-γ receptor, obtained as described in Example 4, at concentration of 20 µM is immobilized on the surface of a streptavidin-coated CM4-Biacore™ sensor chip. For the binding assay, anti-gamma mutant proteins K88Q_T27Y, K88Q_D41N, K88Q and standard (reference) hIFNγ are diluted in HBS-P buffer (10 mM HEPES, pH 7.4, 150 mM NaCl, 0.005% (v/v) surfactant P20) to the desired concentration and are injected on the surface of the IFNGR1-activated sensor chip. Different concentrations of the analytes (0.9, 1.8, 3.6, 7.2 and 15 nM) are tested and their binding to the receptor is measured in arbitrary response units (RU) as a function of time. The experimental data are processed by a Biacore™ software to obtain information about the kinetics of intermolecular interactions (i.e. the ligand-receptor complex formation).

According to the results presented in Table 2, the binding affinity of the single mutant K88Q to the IFNGR1 is comparable with that of the standard hIFN-γ, whereas the K_{A} values for K88Q_T27Y and K88Q_D41N are greater than that of the reference hIFN-γ. The results obtained with SPR support those of iTC and confirm the above conclusion that the two anti-gamma mutant proteins K88Q_T27Y and K88Q_D41N are effective competitors of the wild type hIFN-γ for its receptor.

### REFERENCES

1. Vartanian, V., Li, Y., Zhao, M , Stefansson, K. (1995) Interferon-gemma-induced oligodendrocyte cell death: implications for the pathogenesis of multiple sclerosis. Mol. Med. 1, 732-743.
2. Tellides, G., Pober, J (2007) Interferon-y axis in graft arteriosclerosis. Circ. Res. 100, 622-632
3. Beck, J., Rondot, P., Catinot, L., Falcoff, E., Kirchner, H., Wietzerbin, J. (1988) Increased production of interferon gamma and tumor necrosis factor precedes clinical manifestation in multiple sclerosis: do cytokines trigger off exacerbations? Acta Neurol. Scand. 78, 318-323.
4. Rep, M.H., Hitzen, R. Q., Polman, C.H., van-Lier, R.A. (1996) Recombinant interferon-beta blocks proliferation but enhances interleukin-10 secretion by activated human T-cells. Neuroimmunol. 67, 111-118.
5. Heystek, H C., den Drijver, B., Kapsenberg, M. L., van Lier, R. A., de Jong, E.C. (2003) Type I IFNs differentially modulate IL-12p70 production by human dendritic cells depending on the maturation status of the cells and counteract IFN-gamma-mediated signaling. Clin. Immunol. 107, 170-177.
6. Furlan, R., Bergamim A., Lang, R., Brambilla, E., Franciotta, D., Martinelli, V., Comi, G., Paninam P., Martino. G (2000) Interferon-beta treatment in multiple sclerosis patients decreases the number of circulating T cells producing interferon-gamma and interleukin-4. J. Neuroimmunol. 111, 86-92.
7. Khademi, M., Wallstrom, E., Andersson, M., Piehl, F., Di Marco, R., Olsson, T. (2000) Reduction of both pro- and anti-inflammatory cytokines after 6 months of interferon beta-1a treatment of multiple sclerosis. J. Neuroimmunol 103, 202-210.
8. Skurkovich, S., Boiko, A, Beliaeva, I., Buglak, A., Alekseeva, T., Smirnova, N., Kulakova, O., Tchechonin, V., Gurova, O., Deomina, T., Favorova, O.O., Skurkovic, B., Gusev, E. (2001) Randomized study of antibodies to IFN-gamma and TNF-alpha in secondary progressive multiple sclerosis. Mult. Scler. 7, 277-284.
9. Skurkovich, B., Skurkovich, S (2003) Anti-interferon-gamma antibodies in the treatment of autoimmune diseases Curr. Opin. Mol. Ther. 5, 52-57.
10. Nacheva, G., Todorova, K., Boycheva, M., Berzal-Herranz, A., Karshikiv, A., Ivanov, I. (2003) Human interferon-gamma. significance of the C-terminal flexible domain for its biological activity Arch. Biochem. Biophys. 413, 91-98.
11. Ealick, S.E., Cook, W.J., Vijay-Kumar, S, Carson, M., Nagabhushan, T.L., Trotta, P.P., Bugg, C.E. (1991) Three-dimensional structure of recombinant human interferon-gamma Science 252, 698-702
12. Walter, M.R., Windsor, W.T., Nagabhushan, T.L., Lundell, D.J., Lunn, C.A, Zauodny, P.J., Narula, S K. (1995) Crystal structure of a complex between interferon-gamma and its soluble high-affinity receptor. Nature 376, 230-235.
13. E Lilkova, P. Petkov, N. Ilieva, and L. Litov, Towards molecular modeling of the impact of heparin-derived oligosaccharides on hIFN-γ binding, AIP Conf. Proc. 1684 (2015) 030008. doi: 10 1063/1.4934292
14. Crommelin, D.J.A., Sindelar, R.D , Meibohm, B. (2013) Pharmaceutical biotechnology: fundamentals and applications. Fourth edition. Springer. doi: 10.1007/978-1-4614-6486-0.
15. Tileva, M., Krachmarova, E., Ivanov, I., Maskos, K., Nacheva, G. (2016) Production of aggregation prone human Interferon gamma and its mutant in highly soluble and biologically active form by SUMO fusion technology Protein Expr Purif doi. 10 1016/j.pep 2015.09.022.
16. Boyanova, M., Tsanev, R. and Ivanov, I. (2002) A modified kynurenine bioassay for quantitative determination of human interferon gamma. Analyt. Biochem. 308, 178-181.

## Claims

1. Anti- gamma mutant protein against endogenous human gamma-interferon (hIFN-γ) which is an inactive hIFN-gamma structural analogue with preserved hIFN-gamma receptor affinity where the last five C-terminal amino acid residues of the hIFN-gamma are removed and the amino acid at position 88 is replaced with glutamine (Q), **characterized in that** it represents a derivative of hIFN-γ wherein additionally the amino acid at position 27 is replaced with tyrosine (Y) or the amino acid at position 41 is replaced with asparagine (N).

2. Anti-gamma mutant protein according to claim 1, **characterized in that** it represents a derivative of hIFN-γ, wherein the amino acids at positions 88 and 27 are replaced with glutamine (Q) and tyrosine (Y), respectively.

3. Anti-gamma mutant protein according to claim 1, **characterized in that** it represents a derivative of hIFN-γ, wherein the amino acids at positions 88 and 41 are replaced with glutamine (Q) and asparagine (N), respectively.

4. Anti-gamma mutant protein according to Claims 1-3 for use in the treatment of diseases associated with increased production of endogenous hIFN- gamma.

## Patentansprüche

1. Mutiertes Anti-gamma-Protein gegen endogenes humanes gamma-Interferon (hIFN-γ), wobei die letzten fünf C-terminalen Aminosäurereste entfernt sind und die Aminosäure an Position 88 durch Glutamin (Q) ersetzt ist, **dadurch gekennzeichnet, dass** es sich um ein Derivat von hIFN-γ handelt, wobei zusätzlich die Aminosäure an Position 27 durch Tyrosin (Y) ersetzt ist oder die Aminosäure an Position 41 durch Asparagin (N) ersetzt ist.

2. Mutiertes Anti-gamma-Protein nach Anspruch 1, **dadurch gekennzeichnet, dass** es sich um ein Derivat von hIFN-γ handelt, wobei die Aminosäuren an den Positionen 88 und 27 durch Glutamin (Q) bzw. Tyrosin (Y) ersetzt sind.

3. Mutiertes Anti-gamma-Protein nach Anspruch 1, **dadurch gekennzeichnet, dass** es sich um ein Derivat von hIFN-γ handelt, wobei die Aminosäuren an den Positionen 88 und 41 durch Glutamin (Q) bzw. Asparagin (N) ersetzt sind.

4. Mutiertes Anti-gamma-Protein nach den Ansprüchen 1-3, zur Verwendung bei der Behandlung von Erkrankungen, die mit einer erhöhten Produktion von endogenem hIFN-gamma verbunden sind.

## Revendications

1. Protéine anti-γ mutante dirigée contre l'interféron-γ humain (hIFN-γ) endogène dans laquelle au moins cinq résidus d'acides aminés C-terminaux sont retirés et l'acide aminé à la position 88 est remplacé par la glutamine (Q), **caractérisée en ce qu'**elle représente un dérivé de l'hIFN-γ dans laquelle en outre l'acide aminé à la position 27 est remplacé par une tyrosine (Y) ou l'acide aminé à la position 41 est remplacé par une asparagine (N).

2. Protéine anti-γ mutante selon la revendication 1, **caractérisée en ce qu'**elle représente un dérivé de l'hIFN-γ, dans laquelle les acides aminés aux positions 88 et 27 sont remplacés par la glutamine (Q) et la tyrosine (Y), respectivement.

3. Protéine anti-γ mutante selon la revendication 1, **caractérisée en ce qu'**elle représente un dérivé de l'hIFN-γ, dans laquelle les acides aminés aux positions 88 et 41 sont remplacés par la glutamine (Q) et l'asparagine (N), respectivement.

4. Protéine anti-γ mutante selon l'une quelconque des revendications 1 à 3 pour son utilisation dans le traitement de maladies associées à une production accrue de l'hIFN-γ endogène.


### SEQUENCE LISTING

<110> TIGO GmbH
<120> Anti Gamma Protein
<130> BG
<160> 2
<170> BiSSAP 1.3.6
<210> 1
   <211> 138
   <212> PRT
   <213> mol_type
<220>
   <223> hIFN-gamma
<400> 1
   Gln Asp Pro Tyr Val Lys Glu Ala Glu Asn Leu Lys Lys Tyr Phe Asn
   1               5                   10                  15
   Ala Gly His Ser Asp Val Ala Asp Asn Gly Tyr Leu Phe Leu Gly Ile
               20                  25                  30
   Leu Lys Asn Trp Lys Glu Glu Ser Asp Arg Lys Ile Met Gln Ser Gln
           35                  40                  45
   Ile Val Ser Phe Tyr Phe Lys Leu Phe Lys Asn Phe Lys Asp Asp Gln
       50                  55                  60
   Ser Ile Gln Lys Ser Val Glu Thr Ile Lys Glu Asp Met Asn Val Lys
   65                  70                  75                  80
   Phe Phe Asn Ser Asn Lys Lys Gln Arg Asp Asp Phe Glu Lys Leu Thr
                   85                  90                  95
   Asn Tyr Ser Val Thr Asp Leu Asn Val Gln Arg Lys Ala Ile His Glu
               100                 105                 110
   Leu Ile Gln Val Met Ala Glu Leu Ser Pro Ala Ala Lys Thr Gly Lys
           115                 120                 125
   Arg Lys Arg Ser Gln Met Leu Phe Arg Gly
       130                 135
<210> 2
   <211> 138
   <212> PRT
   <213> mol_type
<220>
   <223> hIFN-gamma
<400> 2
   Gln Asp Pro Tyr Val Lys Glu Ala Glu Asn Leu Lys Lys Tyr Phe Asn
   1 5 10 15
   Ala Gly His Ser Asp Val Ala Asp Asn Gly Thr Leu Phe Leu Gly Ile
   20 25 30
   Leu Lys Asn Trp Lys Glu Glu Ser Asn Arg Lys Ile Met Gln Ser Gln
   35 40 45
   Ile Val Ser Phe Tyr Phe Lys Leu Phe Lys Asn Phe Lys Asp Asp Gln
   50 55 60
   Ser Ile Gln Lys Ser Val Glu Thr Ile Lys Glu Asp Met Asn Val Lys
   65 70 75 80
   Phe Phe Asn Ser Asn Lys Lys Gln Arg Asp Asp Phe Glu Lys Leu Thr
   85 90 95
   Asn Tyr Ser Val Thr Asp Leu Asn Val Gln Arg Lys Ala Ile His Glu
   100 105 110
   Leu Ile Gln Val Met Ala Glu Leu Ser Pro Ala Ala Lys Thr Gly Lys
   115 120 125
   Arg Lys Arg Ser Gln Met Leu Phe Arg Gly
   130 135